# FASCICULE DE BREVET EUROPEEN

(11) **EP 2 380 014 B1**
(45) Date de publication et mention de la délivrance du brevet: **20.09.2017**
(21) Numéro de dépôt: 09806112.0
(22) Date de dépôt: 28.12.2009
(51) Int. Cl.: G01N 27/90, G01M 17/02

(54) **DISPOSITIF DE CONTROLE DE FILS METALLIQUES**
VORRICHTUNG ZUR ÜBERWACHUNG VON METALLDRÄHTEN
DEVICE FOR MONITORING METAL WIRES

(30) Priorité: 31.12.2008 FR 0859166
(43) Date de publication de la demande: 26.10.2011
(73) Titulaire: Compagnie Générale des Etablissements Michelin, 63000 Clermont-Ferrand (FR)
(72) Inventeur: DARDELIN, Thierry, F-63800 Cournon D'auvergne (FR); ALCALDE, Patrice, Simpsonville SC 29681 (US); DECITRE, Jean-Marc, F-91460 Marcoussis (FR); CASULA, Olivier, F-91310 Longpont-sur-orge (FR)
(74) Mandataire: Roussy, Delphine
(86) Numéro de dépôt international: PCT/FR2009/052709
(87) Numéro de publication internationale: WO 2010/076532

(56) Documents cités:
- EP-A1- 0 681 181
- WO-A1-2004/055490
- GB-A- 2 324 155
- US-A- 4 004 693
- US-A- 5 570 017
- US-A- 6 005 388

## Description

La présente invention concerte le domaine technique des pneumatiques.

Un pneumatique comprend plusieurs profilés de gomme superposés les uns aux autres et des fils de renfort noyés dans la gomme de certains de ces profilés. Le terme « fil » doit ici être compris dans un sens général. Ainsi, dans la suite, on désignera, par exemple, par fil un fil monofilament, un fil multifilaments, un câble ou un retors. Le fil pourra être traité en surface ou non. Ces fils forment des nappes et sont disposés sensiblement parallèlement entre eux au sein d'une même nappe. Les fils permettent: de renforcer le pneumatique afin d'en améliorer la résistance et la robustesse. Certains de ces fils sont réalisés dans un matériau magnétique, par exemple en acier, notamment dans les pneumatiques pour véhicule de type poids lourd. Les fils noyés forment des nappes.

Pour contrôler l'état d'un pneumatique, notamment lorsqu'il est usagé, il est recommandé de contrôler chaque fil de renfort afin de s'assurer qu'aucun d'entre eux ne comporte de défaut qui compromettrait l'intégrité du pneumatique. Le terme « défaut » doit ici être compris comme faisant référence à une irrégularité dans le fil. Cette irrégularité peut être notamment une coupure, un détoronnage ou bien encore une diminution significative de section transversale du fil due à l'usure, la corrosion ou l'oxydation du fil.

On connaît de l'état de la technique une installation de contrôle utilisant des rayons X. Toutefois, une telle installation est relativement coûteuse et nécessite des investissements importants.

On connaît également du document US 6,005,388 un dispositif de contrôle du pneumatique. Le dispositif comprend un organe de réception d'un flux magnétique généré à partir de deux organes d'induction. Le flux magnétique génère, au sein de l'organe de réception, un signal d'un état représentatif des fils se trouvant en regard de l'organe de réception. Toutefois, un tel dispositif permet d'identifier grossièrement une zone comprenant plusieurs fils comprenant éventuellement des fils défectueux, sans permettre d'identifier précisément quels fils sont défectueux.

L'invention a pour but de permettre le contrôle et/ou la détection d'éventuels défauts dans chaque fil du pneumatique ainsi que l'identification des éventuels fils défectueux et ce, pour un coût relativement faible. L'invention a également pour but de permettre le contrôle de nappes dont les fils forment des angles différents par rapport à la direction circonférentielle du pneumatique.

A cet effet, l'invention a pour objet un ensemble d'un pneumatique comprenant un fil de renfort de diamètre déterminé réalisé dans un matériau magnétique et d'un dispositif de contrôle de ce fil de renfort, caractérisé en ce que le dispositif comprend :
- au moins un organe d'induction d'un flux magnétique dans le fil à contrôler,
- un support en matériau magnétique portant l'organe d'induction, le support présentant une forme générale en U et comportant des première et deuxième branches de conduction du flux magnétique dans le fil à contrôler, les extrémités de conduction du flux
- un support en matériau magnétique portant l'organe d'induction, le support présentant une forme générale en U et comportant des première et deuxième branches de conduction du flux magnétique dans le fil à contrôler, les extrémités de conduction du flux magnétique dans le fil à contrôler des première et deuxième branches définissant une direction d'alignement du dispositif,
- au moins un organe de réception du flux magnétique circulant dans le fil à contrôler situé entre les première et deuxième branches,
dispositif dans lequel les dimensions de l'organe de réception sont agencées de façon à mesurer seulement le flux magnétique circulant dans le fil à contrôler, lorsqu'en fonctionnement, la direction d'alignement du dispositif est sensiblement parallèle à la direction selon laquelle s'étend le fil à contrôler.

En d'autres termes, les dimensions de l'organe de réception sont agençées de façon à mesurer seulement le flux magnétique circulant dans le fil à contrôler, lorsqu'en fonctionnement, l'alignement du dispositif est orienté parralèllement à la direction dudit fil. L'ensemble selon l'invention permet de détecter un éventuel défaut fil par fil et de localiser avec précision le défaut contrairement au dispositif de l'état de la technique. Il est ainsi possible de dénombrer et d'identifier précisément chaque fil présentant un défaut. En effet, la forme générale en U permet d'orienter le dispositif selon l'invention de sorte que la direction d'alignement est orientée sensiblement suivant la direction du fil à contrôler. Le flux magnétique généré par l'organe d'induction parcourt uniquement le fil situé au droit des branches. Ainsi, il est possible de contrôler ce seul fil au moyen de l'organe de réception qui, en étant situé entre les branches, détecte une éventuelle fuite magnétique sans que les autres fils adjacents au fil contrôlé ne perturbent la détection.

On observera que plus la dimension de l'organe de réception est petite en regard du diamètre du fil à contrôler, meilleure sera la précision de la localisation du fil défaillant.

Les dimensions du ou des organes d'induction peuvent être plus importantes que les dimensions de l'organe de réception. Les organes d'induction peuvent ainsi couvrir ou "éclairer" plusieurs fils, par exemple 3 à 4 fils, sans que cela n'empêche le contrôle fil à fil. En effet, du fait de l'agencement du dispositif en fonctionnement, l'organe de réception va détecter l'éventuelle fuite de flux du seul fil au droit duquel il est situé sans détecter pour autant les éventuelles fuites de flux des autres fils au droit desquels il n'est pas situé.

Dans le cas où une branche viendrait à être au droit d'un fil adjacent présentant une direction différente de celle du fil contrôlé, le flux circulant dans ce fil adjacent ne serait pas fermé par l'autre branche, si bien qu'aucun flux parasite provenant de ce fil adjacent n'est susceptible de perturber le contrôle du fil que l'on cherche à contrôler.

Le contrôle fil à fil repose donc sur le principe physique de l'induction d'un flux magnétique dans chaque fil par au moins une des branches et la détection de ce flux par l'organe de réception du ou des flux.

En outre, plus les branches sont rapprochées, plus la zone contrôlée est réduite. Ainsi, on peut facilement localiser un défaut dans chaque fil par déplacement du dispositif le long de chaque fil. La tête de l'organe de réception présente par exemple des dimensions pertinentes par rapport à la distance ou au pas séparant deux fils contigus à contrôler. Le dispositif détecte donc la présence d'un éventuel défaut sur un fil, indépendamment de la présence ou non d'un défaut sur un des fils contigus au fil contrôlé.

En outre, un tel ensemble présente l'avantage de permettre un contrôle non destructif du pneumatique ou de la nappe de renfort.

De préférence, l'organe de réception comprend une tête de réception du flux présentant, dans la direction perpendiculaire à la direction d'alignement, une dimension sensiblement inférieure ou égale au diamètre du fil. Par la largeur de la tête de réception, on fait référence à la dimension de la partie formant capteur de l'organe de réception. Cette partie est spécifique à chaque type de capteur. La tête de réception ayant une dimension inférieure ou égale au diamètre du fil, le signal de détection sera donc maximum lorsqu'il sera placé au-dessus du fil à contrôler, étant entendu que les fils ne sont pas en contact les uns des autres et sont séparés par un espace.

Avantageusement, la dimension de la tête de réception est inférieure ou égale à 5 mm, voire 3 mm et de préférence 2 mm.

D'une manière générale, les fils à contrôler présentent un diamètre inférieur à 5 mm et la plupart d'entre eux ont un diamètre variant entre 1 à 2 mm, et sont espacés les uns des autres d'un pas d'au moins 1 mm. Aussi, en pratique, la dimension de la tête de réception peut utilement être comprise entre 2 et 3 mm, de manière à permettre le contrôle d'une large variété de pneumatiques.

De préférence, l'organe de détection est situé à équidistance des première et deuxième branches ce qui améliore le rapport signal/bruit.

Dans un mode de réalisation, l'organe de réception est classiquement de type inductif et comporte une bobine réceptrice. Il comprend un capteur magnétique du type magnéto-résistance, magnéto-impédance ou à effet Hall. De façon optionnelle, l'organe de réception peut comprendre un capteur magnétique tel qu'une magnéto-résistance anisotrope, une magnéto-résistance géante ou une magnéto-impédance géante.

L'organe d'induction quant à lui, peut être de type inductif alimenté par une source de courant alternatif ou continu. De façon optionnelle, si l'organe de réception fonctionne sur un mode statique, il peut être constitué par un ou plusieurs aimants permanents. signal dans l'organe de réception.

Le signal peut-être par exemple, et suivant le type d'organe de détection, une force électromotrice, une tension électrique ou une variation de résistance.

Les premier et deuxième organes d'induction créent avantageusement chacun un flux magnétique d'intensité semblable et de sens tels que le flux s'annule au moins partiellement au niveau de l'organe de réception.

Dans le cas d'une force électromotrice et en présence d'un défaut, le dispositif permet de détecter une variation de la force électromotrice mesurée par rapport à une valeur de référence de la force électromotrice.

Selon d'autres caractéristiques optionnelles :
- Le dispositif est agencé de sorte que chaque premier et deuxième flux magnétique est apte à circuler respectivement dans une première et une deuxième partie du fil située entre l'organe de réception et respectivement la première et la deuxième branche.
- Chaque premier et deuxième organe d'induction comprend respectivement des première et deuxième bobines d'induction, chaque première et deuxième bobine d'induction étant agencée autour respectivement des première et deuxième branches.
- L'organe de réception comprend un noyau autour duquel est agencée une bobine de réception des premier et deuxième flux électromagnétiques, le noyau formant la tête de réception des premier et deuxième flux magnétiques.

L'invention a également pour objet un procédé de contrôle d'un pneumatique comprenant des fils de renfort de diamètre déterminé réalisés dans un matériau magnétique ou dune nappe comprenant de tels fils de renfort et
disposés sensiblement parallèlement entre eux en faisant un angle donné avec la direction circonférentielle du pneumatique ou avec la direction longitudinale de la nappe au moyen d'un ensemble selon l'une quelconque des revendications 1 à 8, **caractérisé en ce qu**'on contrôle successivement chaque fil du pneumatique ou de la nappe indépendamment les uns des autres.

Le procédé selon l'invention permet de contrôler l'état de chaque fil indépendamment de l'état des fils contigus au fil contrôlé. Ainsi, on peut identifier précisément quel fil présente un défaut.

Ce procédé peut s'appliquer à une ébauche crue de pneumatique, un pneumatique vulcanisé neuf, un pneumatique rechapé ou bien encore à une nappe de gomme.

Avantageusement, on maintient l'organe de réception au contact du pneumatique ou de la nappe à contrôler.

Le procédé selon l'invention permet de contrôler l'état de chaque fil indépendamment de l'état des fils contigus au fil contrôlé. Ainsi, on peut identifier précisément quel fil présente un défaut.

Ce procédé peut s'appliquer à une ébauche crue de pneumatique, un pneumatique vulcanisé neuf, un pneumatique rechapé ou bien encore à une nappe de gomme.

Avantageusement, on maintient l'organe de réception au contact du pneumatique ou de la nappe à contrôler.

De même, il s'avère intéressant de maintenir le ou chaque organe d'induction au contact du pneumatique ou de la nappe à contrôler.

De préférence, l'organe de réception comprend une tête de réception du flux présentant, dans la direction perpendiculaire à la direction d'alignement, une dimension sensiblement inférieure ou égale au diamètre du fil.

Selon une caractéristique optionnelle, on déplace le pneumatique ou la nappe par rapport au dispositif et on mesure un signal représentatif de l'état du ou de chaque fil en fonction du déplacement relatif du pneumatique ou de la nappe par rapport au dispositif.

Dans un mode de réalisation, on compare la valeur du signal mesuré à une valeur de référence de sorte qu'on détecte un éventuel défaut du ou de chaque fil.

Dans un autre mode de réalisation, on détermine une caractéristique géométrique du ou de chaque fil en fonction d'une variation du signal mesuré.

Dans une variante :
- on déplace circonférentiellement le pneumatique ou la nappe dans une direction longitudinale, par rapport au dispositif respectivement sur une longueur circonférentielle donnée ou une longueur donnée,
- on mesure la variation du signal en fonction du déplacement du pneumatique ou de la nappe,
- on détermine :
   o un nombre de fils dans la longueur circonférentielle du pneumatique ou la longueur de la nappe, ou/et
   o un pas entre chaque fil dans la longueur circonférentielle du pneumatique ou la longueur de la nappe.

Généralement, le pneumatique comprend des fils régulièrement espacés. Le signal varie donc de façon périodique. Selon les réglages du dispositif, la présence d'un fil correspond, par exemple, à un minima ou un maxima du signal. On en déduit donc le nombre de fils dans la longueur circonférentielle de pneumatique ou la longueur de nappe et, en connaissant la vitesse de déplacement relatif entre le pneumatique ou la nappe et le dispositif, le pas entre chaque fil dans la longueur circonférentielle de pneumatique ou la longueur de nappe.

Dans une autre variante :
- on déplace en rotation le dispositif autour d'un axe sensiblement perpendiculaire à une surface, dite circonférentielle, du pneumatique ou une surface, dite longitudinale, de la nappe,
- on mesure la variation du signal en fonction de l'angle de rotation du dispositif,
- on détermine l'angle formé par chaque fil avec la direction circonférentielle du pneumatique ou avec la direction longitudinale de la nappe.

Selon les réglages du dispositif, la présence d'un fil correspond, par exemple, à un minima ou un maxima du signal lorsque le dispositif est orienté sensiblement parallèlement au fil, c'est-à-dire lorsque la direction d'alignement est sensiblement parallèle au fil à contrôler. Ainsi, lorsqu'on met en rotation le dispositif, le signal varie jusqu'à atteindre un extrema, par exemple un maximum, lorsque l'angle de rotation du dispositif est sensiblement égal à l'angle formé par le fil avec la direction longitudinale de la nappe ou avec la direction circonférentielle du pneumatique. Le document GB2324155 divulgue un ensemble d'un pneumatique comprenant un fil de renfort de diamètre déterminé réalisé dans un matériau magnétique et d'un dispositif de contrôle de ce fil de renfort comprenant : - au moins un organe d'induction d'un flux magnétique dans le fil à contrôler, - un support en matériau magnétique portant l'organe d'induction, le support présentant une forme générale en U et comportant des première et deuxième branches de conduction du flux magnétique dans le fil à contrôler, - plusieurs organes de réception du flux magnétique circulant dans le fil à contrôler situé du côté opposé à celui des première et deuxième branches dudit support. Enfin le document US 5,570,017 décrit un dispositif de contrôle d'un fil de renfort de diamètre déterminé réalisé dans un matériau magnétique, lequel fil de renfort appartient à un tapis de convoyeur. L'invention sera mieux comprise à la lecture de la description qui va suivre, uniquement à titre d'exemple non limitatif et faite en se référant aux dessins dans lesquels :
- la figure 1 est une vue en perspective d'une installation de contrôle comprenant un ensemble d'un pneumatique et dispositif de contrôle selon un premier mode de réalisation permettant la mise en oeuvre d'un procédé de contrôle selon un premier mode de réalisation;
- la figure 2 est une vue de profil d'une partie de l'installation de la figure 1 ;
- la figure 3 est une vue en coupe de l'installation de la figure 1 ;
- les figures 4 et 5 sont des schémas illustratifs d'un dispositif de l'installation de la figure 1 ;
- la figure 6 est une vue analogue à la figure 1 d'une installation comprenant un ensemble selon un deuxième mode de réalisation permettant la mise en oeuvre d'un procédé de contrôle selon un deuxième mode de réalisation ;
- la figure 7 est une vue analogue à la figure 3 de l'installation de la figure 6 ;
- les figures 8 et 9 illustrent schématiquement respectivement des installations comprenant des ensembles selon des troisième et quatrième modes de réalisation permettant la mise en oeuvre de procédés de contrôle selon des troisième et quatrième modes de réalisation ;
- la figure 10 est une vue en perspective d'une installation selon un cinquième mode de réalisation permettant la mise en oeuvre d'un procédé de contrôle selon un cinquième mode de réalisation ;
- la figure 11 illustre des moyens d'articulation de l'ensemble d'un pneumatique et du dispositif de contrôle de l'installation de la figure 10 ;
- la figure 12 est une vue de dessus de l'installation de la figure 10 ;
- les figures 13 et 14 illustrent des procédés de contrôle selon des sixième et septième modes de réalisation ;
- la figure 15 illustre un dispositif selon un huitième mode de réalisation.

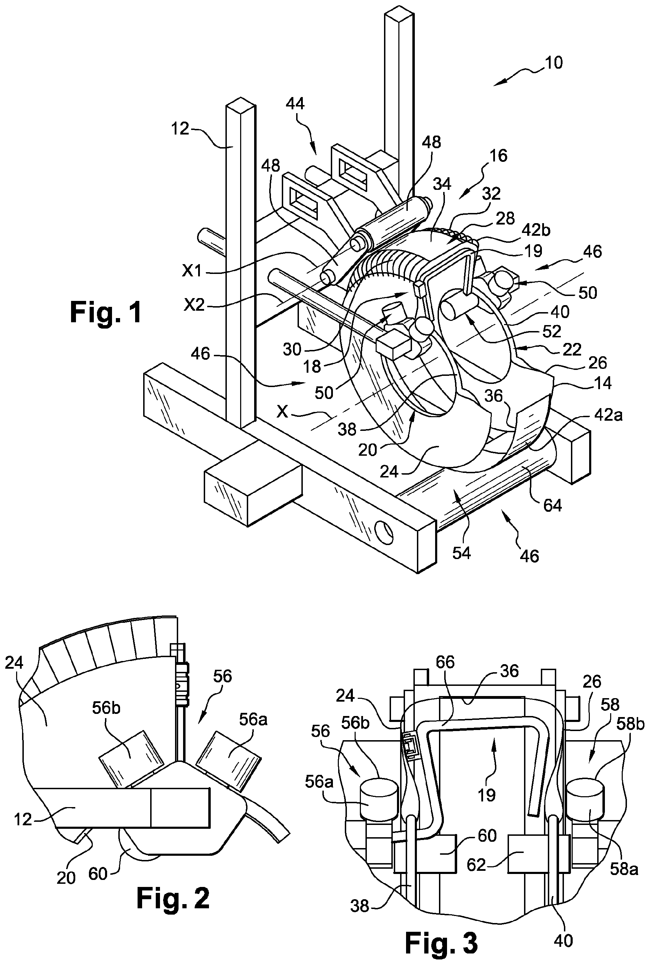

On a représenté sur les figures 1 à 3 une installation comprenant ensemble d'un pneumatique et dispositif de contrôle selon un premier mode de réalisation et désignée par la référence générale 10. L'installation 10 comprend un bâti 12 de support d'un pneumatique 14. En variante, le bâti 12 pourra supporter une ébauche crue du pneumatique 14. L'installation 10 comprend également des moyens 16 de mise en rotation du pneumatique 14. L'installation 10 comprend un dispositif 18 de contrôle du pneumatique 14 ainsi que des moyens 19 de guidage du dispositif 18.

Le pneumatique 14 est sensiblement de révolution autour d'un axe de révolution X. Le pneumatique 14 comporte des premier et deuxième bords 20, 22 d'accroche radialement interne le délimitant. Le pneumatique 14 comprend des premier et deuxième flancs 24, 26 ainsi qu'une bande de roulement 28. La bande de roulement 28 est délimitée axialement par des première et deuxième extrémités 30, 32, appelées épaules.

Le pneumatique 14 comporte de plus une surface radialement externe 34, dite circonférentielle, de roulement et une surface radialement interne 36. Le pneumatique 14 comprend également des première et deuxième tringles circulaires 38, 40 sensiblement coaxiales à l'axe X. Enfin, le pneumatique 14 comprend des fils 42a, 42b de renfort réalisés dans un matériau magnétique, en l'espèce métallique, ici en acier. Les fils de renfort 42a situés sous la bande de roulement 28 forment la ceinture de renfort sommet. Les fils de renfort 42b s'étendant entre les première et deuxième tringles 38, 40 forment la nappe de renfort carcasse. Les fils 42a, 42b sont regroupés dans différentes nappes et sont disposés sensiblement parallèlement entre eux au sin d'une même nappe.

Les moyens de mise en rotation 16 comprennent des moyens 44 d'entraînement du pneumatique 14 ainsi que des moyens 46 de guidage du pneumatique. Les moyens 44 comprennent deux rouleaux 48 motorisés en rotation autour d'axes X1, X2 sensiblement parallèles à l'axe X et positionnés au contact de la bande de roulement 28 de façon à entraîner le pneumatique 14 en rotation. Les moyens 42 comprennent également des moyens 50, 52, 54 de guidage axial, radial et circonférentiel du pneumatique 14. Les moyens de guidage axial 50 comprennent des première et deuxième paires 56, 58 de galets 56a, 56b et 58a, 58b de guidage. Les première et deuxième paires 56, 58 sont disposées sensiblement symétriquement par rapport à un plan axial médian du pneumatique 14. Les galets 56a-b, 58a-b sont respectivement positionnés au contact des premier et deuxième flancs 24, 26. Les moyens de guidage radial 52 comprennent des premier et deuxième galets 60, 62 de guidage positionnés respectivement au contact des premier et deuxième bord 20, 22. Les moyens de guidage circonférentiel 54 comprennent un rouleau 64 de roulage du pneumatique 14 libre en rotation autour d'un axe X3 sensiblement parallèle à l'axe X.

Les moyens de guidage 19 du dispositif 18 sont agencés de sorte que le dispositif 18 suive le contour de la surface radialement interne 36 lorsque le dispositif 18 est déplacé le long des moyens de guidage 19. Les moyens de guidage 19 comprennent un guide formant rail 66. Le guide 66 présente une forme générale en U inversé.

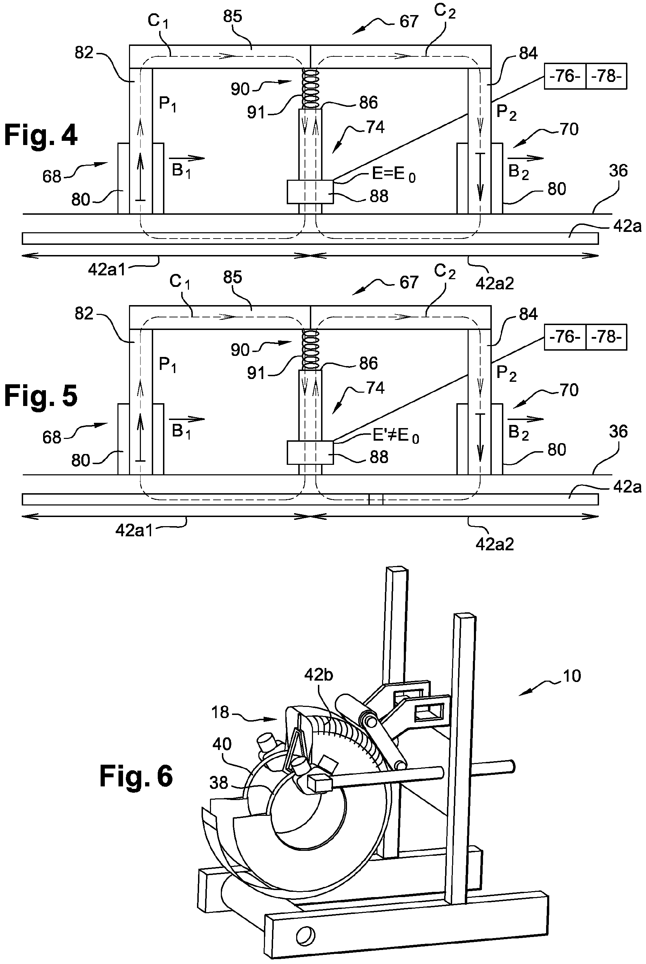

En référence aux figures 4 et 5, le dispositif 18 est du type à courant de Foucault. Le dispositif 18 comprend un support 67 en matériau magnétique portant des premier et deuxième organes 68, 70 d'induction respectivement de premier et deuxième champs électromagnétiques B1, B2 générant respectivement des premier et deuxième flux magnétiques P1, P2 dans le support 67 et chaque fil 42a, 42b. En outre, le dispositif 18 comprend un organe 74 de réception des premier et deuxième flux P1, P2. L'organe de réception 74 est situé entre les premier et deuxième organes d'induction 68, 70 de sorte que les premier et deuxième champs B1, B2 sont aptes à générer un signal représentatif de l'état de chaque fil 42a, 42b dans l'organe 74. Les champs B1, B2 génèrent un signal dans l'organe de réception 74, et dont la nature dépend du type de récepteur comme cela a été exposé précédemment. Par commodité dans ce qui suit, on considèrera que le signal représentatif en question est une force électromotrice notée E.

L'organe de réception 74 est situé à équidistance des organes 68, 70 et donc des branches 82, 84. Le dispositif 18 est réglé de sorte que les valeurs algébriques des champs B1 et B2 sont telles que la valeur du signal (la force électromotrice E) est minimale, voire nulle, si le fil présente une symétrie, et ne présente aucun défaut. Il est alors possible de jouer sur l'amplitude et la phase des champs B1 et B2 pour faire tendre la valeur du signal (la force électromotrice E) vers une valeur d'amplitude minimale du signal.

En variante, l'organe 74 pourra être plus proche d'un des organes 68, 70 que de l'autre. Le dispositif 18 comprend également des moyens 76 de mesure du signal (la force électromotrice E) ainsi que des moyens 78 de comparaison du signal (la force électromotrice E) avec une valeur de référence (force électromotrice E₀)

Chaque organe d'induction 68, 70 comprend une bobine d'induction 80. Le support 67 présente une forme générale en U comportant des première et deuxième branches magnétiques 82, 84 de conduction respectivement des premier et deuxième flux P1, P2 dans chaque fil 42a, 42b. Le support comprend également une barre transversale 85 reliant entre elles les branches 82, 84. Chaque bobine d'induction 80 est agencée autour de chaque branche 82, 84.

Chaque première et deuxième branche présente une extrémité de conduction de chaque flux magnétique P1, P2 dans le fil à contrôler. Les extrémités définissent une direction d'alignement. Les premier et deuxième organes d'induction 68, 70 et l'organe de réception 74 sont également sensiblement alignés suivant la direction d'alignement. La direction d'alignement est sensiblement parallèle à la direction selon laquelle s'étend chaque fil à contrôler. Ainsi, le dispositif est agencé de façon à mesurer seulement le flux magnétique circulant dans le fil à contrôler lorsque, en fonctionnement, la direction d'alignement est orientée sensiblement parralèllement à la direction selon laquelle s'étend le fil à contrôler.

L'organe de réception 74 comprend une tête de réception 86, en l'espèce un noyau autour duquel est agencée une bobine 88 de réception des flux P1, P2. Les branches 82, 84, la barre transversale 85 et le noyau 86 sont réalisés en ferrite. La tête 86 est destinée à être disposée au droit de la surface 34 du pneumatique 14 et présente des dimensions pertinentes à l'égard de la réception des flux magnétiques P1, P2 de sorte que l'organe de réception est apte à détecter une variation du flux lorsque le dispositif est déplacé par rapport au pneumatique 14 sur une distance égale au pas séparant deux fils 42a, 42b contigus à contrôler. La tête 86 présente une forme sensiblement cylindrique de diamètre sensiblement égal à 1 mm et de longueur environ égale à 33 mm. La bobine 88 comprend plusieurs spires d'un fil enroulé autour du noyau 86. Le fil présente un diamètre inférieur à 1 mm et est égal, en l'espèce, à 0,6 mm. La bobine 88 comporte une centaine de spires, précisément 103 spires réparties sur quatre couches superposées. La bobine 88 s'étend sur une hauteur de 2 mm sur le noyau 86. Dans l'exemple représenté, la fréquence de résonance de l'organe de réception 74 est égale à 960 KHz. En outre, l'organe 74 présente une impédance de 73 Ω à 100 KHz pour une inductance de 115 µH. Une bobine de ce type est apte à détecter une variation de flux lorsque ledit dispositif franchit le pas séparant deux fils contigus à contrôler.

Le dispositif comprend également des moyens 90 de maintien du noyau 86 au contact de la surface 36. Ces moyens 90 comprennent notamment un ressort 91.

En référence aux figures 4 et 5, le fil 42a comprend des première et deuxième parties 42a1, 42a2. La première partie 42a1 est située entre l'organe 74 et l'organe d'induction 68. La deuxième partie 42a2 est située entre l'organe 74 et l'organe d'induction 70. Le dispositif 18 est agencé de sorte que chaque premier et deuxième flux magnétique P1, P2 est apte à circuler respectivement dans la première et la deuxième partie 42a1, 42a2 du fil 42a.

En l'absence de défaut sur le fil 42 (figure 4), les deux flux magnétiques P1, P2 traversant alors l'organe de réception 74 génèrent un signal (une force électromotrice E₀), dit de référence, correspondant à deux circuits magnétiques C1, C2 fermés représentés en pointillés. Dans le cas où les organes d'induction 68, 70 sont strictement identiques, la valeur du signal (la force électromotrice E₀) de référence est égale à 0 lorsque la phase des tensions créant les flux P1 et P2 est correctement ajustée. En variante, les organes 68, 70 présentent des caractéristiques différentes de sorte que la valeur du signal (la force électromotrice E₀) de référence est différente de 0.

En présence d'un défaut sur le fil 42 (figure 5), ici sur la deuxième partie 42a2, les deux flux magnétiques P1, P2 traversant alors l'organe de réception 74 génèrent une force électromotrice E' correspondant, dans l'exemple représenté, à un circuit magnétique C1 fermé, et à un circuit magnétique C2 présentant une reluctance supérieure au circuit magnétique C2 de la figure 4. La valeur du signal mesuré (la force électromotrice E') est différente de la valeur du signal de référence (force électromotrice E₀).

L'installation 10 permet de mettre en oeuvre un procédé de contrôle selon un premier mode de réalisation dont on décrira ci-dessous les principales étapes liées à l'invention.

On positionne le pneumatique 14 dans l'installation 10. On positionne les premier et deuxième bords 20, 22 d'accroche respectivement sur les premier et deuxième galets de guidage 60, 62 et les premier et deuxième flancs 24, 26 respectivement contre les galets 56a-b, 58a-b.

Puis, on plaque les rouleaux 48 contre la surface de roulement 34.

On contrôle alors le pneumatique 14 en vue de détecter un éventuel défaut des fils 42a, 42b du pneumatique 14. On contrôle successivement chaque fil 42a, 42b indépendamment les uns des autres.

Pour ce faire, on positionne les premier et deuxième organes d'induction 68, 70 en regard du pneumatique 14 et on maintient le noyau 86 au contact de la surface radialement interne 36. Les organes d'induction 68, 70 peuvent également être maintenus au contact de ladite surface interne 36.

On met alors en rotation le pneumatique 14 autour de l'axe X au moyen des rouleaux 48 et on mesure la valeur du signal (force électromotrice E) générée dans l'organe de réception 74 en fonction du déplacement relatif du pneumatique 14 par rapport au dispositif 18. On maintient le noyau 86 au contact de la surface interne 36. On fait faire un tour au pneumatique 14. En variante, et afin de fiabiliser la détection d'un éventuel défaut, on fait faire plusieurs tours au pneumatique 14. Lors de cette mesure, on compare chaque valeur du signal (de la force électromotrice E) mesurée pour chaque fil à la valeur du signal de référence (la force électromotrice E₀), de sorte qu'on détecte un éventuel défaut de chaque fil 42a, 42b.

On contrôle ainsi une première portion du pneumatique 14 en regard des premier et deuxième organes d'induction 68, 70. Cette portion forme une bande circulaire dont la largeur est sensiblement égale à la distance entre les premier et deuxième organes d'induction 68, 70.

Puis, on déplace axialement et/ou radialement le dispositif 18 le long du contour de la surface 36 et on met à nouveau en rotation le pneumatique 14 autour de l'axe X. On contrôle ainsi une deuxième bande circulaire, adjacente à la première portion.

Pour chaque portion et lorsqu'on détecte un défaut, on mesure une position du pneumatique 14 autour de l'axe X par rapport à une position de référence. En l'espèce, on détermine la position angulaire du pneumatique 14 par rapport à une position angulaire de référence. Dans le mode de réalisation illustré, on relève également la position du dispositif 18 dans les moyens de guidage 19 lorsqu'on détecte un défaut de façon à déterminer la bande circulaire comprenant le défaut. En procédant par itérations successives, on contrôle entièrement le pneumatique 14.

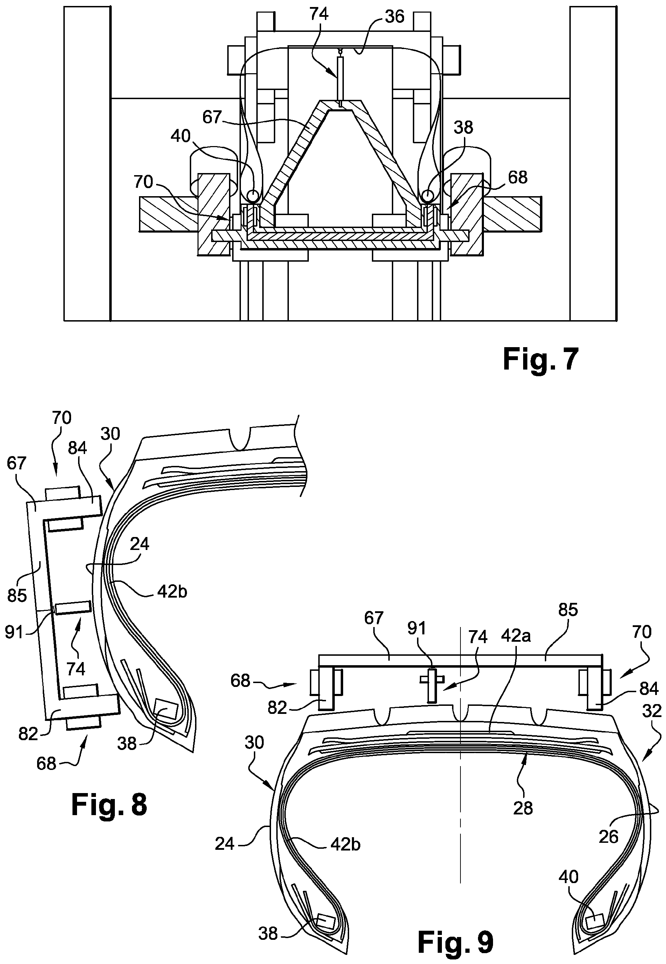

On a représenté sur les figures 6 et 7 une installation selon un deuxième mode de réalisation. Les éléments analogues à ceux représentés sur les figures précédentes sont désignés par des références identiques.

A la différence du premier mode de réalisation, l'installation 10 ne comprend pas de moyens de guidage 19 du dispositif 18. Les premier et deuxième organes d'induction 68, 70 ainsi que l'organe de réception 74 sont fixes axialement et radialement par rapport au pneumatique 14.

Dans ce mode de réalisation, l'organe 74 est positionné sensiblement dans un plan axial médian du pneumatique 14 et la tête 86 est positionnée au contact de la surface interne 36.

L'installation 10 des figures 6 et 7 permet de mettre en oeuvre un procédé de contrôle selon un deuxième mode de réalisation.

Dans ce mode de réalisation, on positionne chaque premier et deuxième organe d'induction 68, 70 axialement sensiblement en regard respectivement de chaque première et deuxième tringle 38, 40. On maintient le noyau 86, au contact de la surface interne 36 de sorte qu'on détecte un éventuel défaut de chaque fil 42b s'étendant entre les première et deuxième tringles 38, 40.

On a représenté sur les figures 8 et 9 une installation selon des troisième et quatrième modes de réalisation. Les éléments analogues à ceux représentés sur les figures précédentes sont désignés par des références identiques.

L'installation 10 de la figure 8 permet de mettre en oeuvre un procédé de contrôle selon un troisième mode de réalisation.

Dans ce mode de réalisation, on positionne axialement le premier organe d'induction en regard de l'extrémité 30, 32 et le deuxième organe d'induction en regard de la tringle 38, 40. On maintient la tête 86 au contact du flanc 24, 26, on met en rotation le pneumatique 14 et on contrôle ainsi respectivement chaque flanc 24, 26, de sorte qu'on détecte un éventuel défaut de chaque fil 42b de la nappe de renfort carcasse situé dans une zone, dite flanc, s'étendant entre l'extrémité 30, 32 et la tringle 38, 40.

L'installation 10 de la figure 9 permet de mettre en oeuvre un procédé de contrôle selon un quatrième mode de réalisation.

Dans ce mode de réalisation, on positionne axialement chaque premier et deuxième organe d'induction sensiblement en regard respectivement de chaque première et deuxième extrémité axiale 30, 32 de la bande de roulement 28. On maintient la tête 86 au contact de la surface externe 34 de la bande de roulement 28 et on met en rotation le pneumatique 14 de sorte qu'on détecte un éventuel défaut de chaque fil 42b d'une des nappes de renfort carcasse.

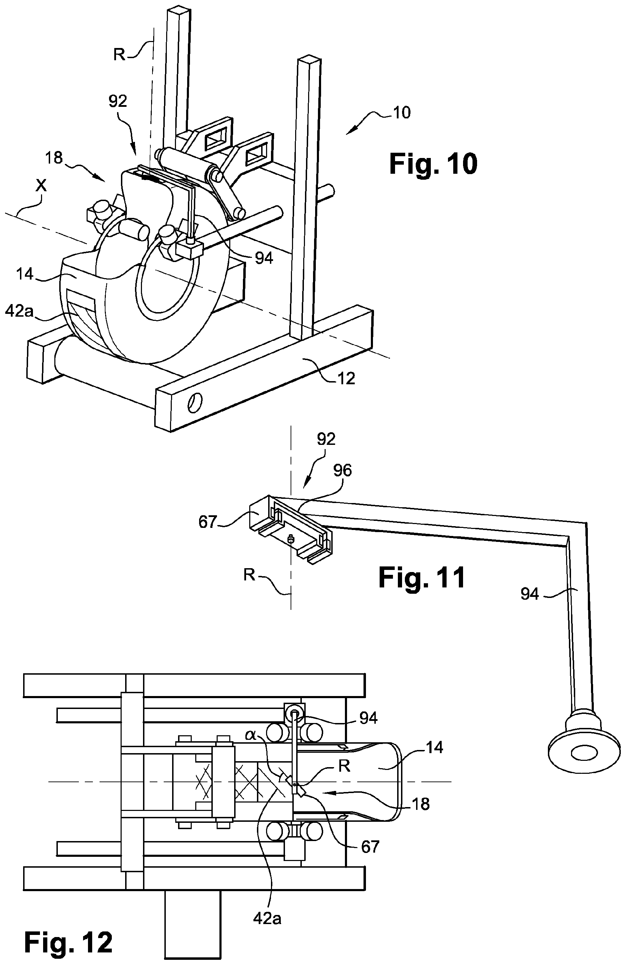

On a représenté sur les figures 10 à 12 une installation selon un cinquième mode de réalisation. Les éléments analogues à ceux représentés sur les figures précédentes sont désignés par des références identiques.

L'installation 10 comprend également des moyens 92 d'articulation du dispositif 18 par rapport au pneumatique 14. Les moyens d'articulation 92 forment des moyens d'articulation en rotation du support 67 autour d'un axe radial R sensiblement perpendiculaire à l'axe de rotation X du pneumatique et normal à la surface circonférentielle 34. Les moyens d'articulation 92 comprennent un bras 94 auquel est fixé le dispositif 18 au moyen d'une liaison rotative 96.

L'installation 10 des figures 10 à 12 permet de mettre en oeuvre un procédé de contrôle selon un cinquième mode de réalisation.

A la différence des modes de réalisation précédents dans lesquels on contrôle les fils 42b de la nappe de renfort carcasse s'étendant suivant une direction sensiblement radiale, on contrôle, dans le cinquième mode de réalisation, les fils 42a des nappes de renfort sommet.

Les fils 42a s'étendent suivant une direction faisant un angle inférieur à 90° par rapport à la direction circonférentielle du pneumatique 14. Dans le cas d'espèce illustré par les figures 10 à 12, cette direction forme un angle ±α de ±45°, avec la direction circonférentielle du pneumatique 14.

On fait pivoter le dispositif 18 autour de l'axe R d'un angle ±α, ici ±45°, et on contrôle le pneumatique 14 d'une manière analogue à celle décrite dans le premier mode de réalisation.

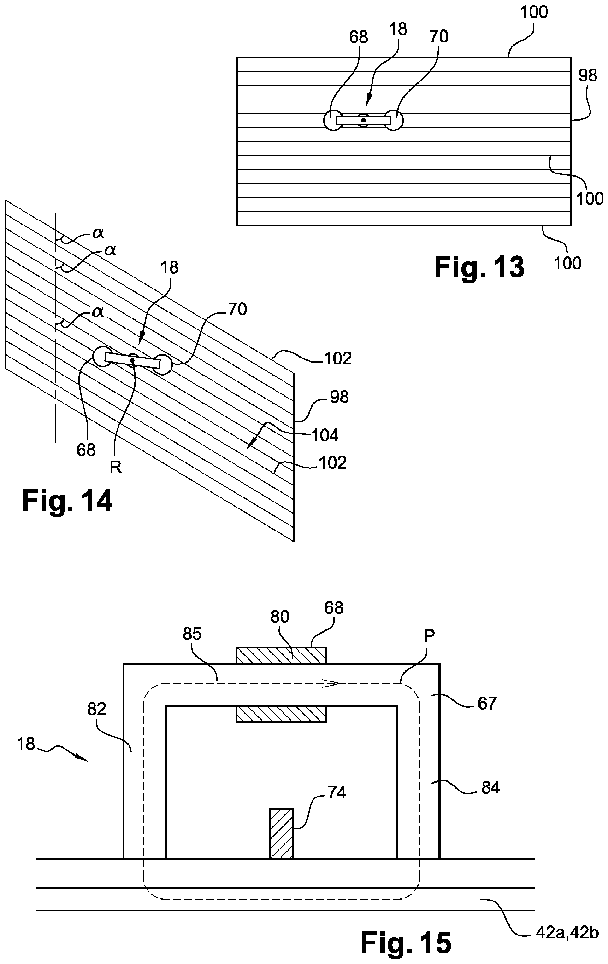

On a représenté de façon schématique sur la figure 13 un dispositif selon un sixième mode de réalisation. Les éléments analogues à ceux représentés sur les figures précédentes sont désignés par des références identiques.

Le dispositif 18 selon le sixième mode de réalisation permet le contrôle d'une nappe de renfort 98. La nappe 98 comprend des fils métalliques 100 enrobés dans un mélange de caoutchouc, disposés sensiblement parallèlement entre eux et formant un angle donné avec la direction longitudinale de la nappe 98. A la différence de l'installation selon le cinquième mode de réalisation, l'installation 10 selon le sixième mode de réalisation ne comprend pas de moyens 16 de mise en rotation du pneumatique 14 mais des moyens de déplacement (non représentés) de la nappe de renfort 98. Les fils 100 sont disposés sensiblement perpendiculairement à la direction longitudinale de la nappe de renfort 98 dans le cas d'une nappe de renfort carcasse comme illustrée à la figure 13.

L'installation 10 de la figure 13 permet de mettre en oeuvre un procédé de contrôle selon un sixième mode de réalisation.

On déplace la nappe de renfort 98 par rapport au dispositif 18. On déplace la nappe de renfort 98 en translation dans une direction sensiblement parallèle à la direction longitudinale de la nappe de renfort 98 sur une longueur donnée L. On mesure une variation de la valeur du signal (de la force électromotrice) en fonction du déplacement de la nappe de renfort 98. Puis, grâce à la connaissance de la vitesse de déplacement de la nappe de renfort 98, on détermine une caractéristique géométrique des fils 100 en fonction de la variation de la valeur du signal (de la force électromotrice) mesurée. En l'espèce, on détermine un nombre de fils 100 dans la longueur de la nappe de renfort 98 déplacée. En variante, on détermine un pas entre les fils 100 dans la longueur de la nappe de renfort 98 déplacée.

En variante, on contrôle un pneumatique 14. Dans cette variante, on fait tourner circonférentiellement autour de l'axe X par rapport au dispositif 18 respectivement sur une longueur circonférentielle donnée et on mesure la variation du signal en fonction du déplacement du pneumatique 14. On détermine ainsi le nombre de fils dans la longueur circonférentielle du pneumatique 14 ou/et le pas entre chaque fil 100 dans la longueur circonférentielle du pneumatique 14.

On a représenté sur la figure 14 un dispositif selon un septième mode de réalisation. Les éléments analogues à ceux représentés sur les figures précédentes sont désignés par des références identiques.

A la différence de la nappe de renfort 98 du sixième mode de réalisation, la nappe de renfort 98 comprend des fils 102 s'étendant selon une direction formant un angle ±α inférieur à 90° avec la direction longitudinale de la nappe, comme dans le cas d'une nappe de renfort sommet telle qu'illustrée à la figure 14. Ici, l'angle α est environ égal à ±45°.

D'une manière analogue à l'installation selon le cinquième mode de réalisation, l'installation 10 selon le septième mode de réalisation comprend des moyens d'articulation (non représentés) en rotation du dispositif 18 par rapport à la nappe de renfort 98 autour d'un axe R sensiblement perpendiculaire à la surface longitudinale 104 de la nappe de renfort 98 et passant par la tête 86.

L'installation 10 de la figure 14 permet également de mettre en oeuvre un procédé de contrôle selon un septième mode de réalisation.

On contrôle successivement l'orientation de chaque fil 102 indépendamment les uns des autres. On déplace en rotation le dispositif 18 autour de l'axe R. On mesure la variation de la valeur du signal (de la force électromotrice) en fonction d'un angle de rotation du dispositif 18 autour de l'axe R. Puis, on détermine l'angle formé par chaque fil 100 avec la direction longitudinale de la nappe 98.

En variante, on contrôle un pneumatique 14. Dans cette variante, on déplace en rotation le dispositif autour de l'axe R sensiblement perpendiculaire à la surface 34 du pneumatique 14 et on mesure la variation du signal en fonction de l'angle de rotation du dispositif. Puis, on détermine l'angle formé par chaque fil 102 avec la direction circonférentielle du pneumatique 14.

On a représenté sur la figure 15 un ensemble selon un huitième mode de réalisation. Les éléments analogues à ceux représentés sur les figures précédentes sont désignés par des références identiques.

Le dispositif 18 comprend un organe 68 d'induction d'un flux magnétique P dans chaque fil 42a, 42b. Le dispositif 18 comprend également un organe 74 de réception du flux magnétique P. Le dispositif 18 comprend également un support en matériau magnétique 67 portant l'organe d'induction 68. Le support 67 présente une forme générale en U et comporte des première et deuxième branches 82, 84 de conduction du flux magnétique P dans chaque fil 42a, 42b. L'organe de réception 74 est situé entre les branches 82, 84 et comprend un capteur magnétique, en l'espèce une magnéto-résistance. A titre d'exemple, il a été utilisé avec succès une magnéto-résistance référence AAH004 de la Société NVE encapsulée dans un boîtier du type MSOP8 fourni par la même Société.

En variante, l'organe 74 comprend un capteur à effet Hall, une magnéto-impédance ou tout autre capteur apte à détecter un champ magnétique.

## Revendications

1. Ensemble d'un pneumatique comprenant un fil de renfort (42a-b ; 100; 102) de diamètre déterminé réalisé dans un matériau magnétique et d'un dispositif (18) de contrôle de ce fil de renfort, **caractérisé en ce que** le dispositif comprend :
- au moins un organe d'induction (68, 70) d'un flux magnétique (P1, P2 ; P) dans le fil à contrôler (42a-b ; 100 ; 102),
- un support (67) en matériau magnétique portant l'organe d'induction (68, 70), le support (67) présentant une forme générale en U et comportant des première et deuxième branches (82, 84) de conduction du flux magnétique (P) dans le fil à contrôler (42a-b ; 100 ; 102), les extrémités de conduction du flux magnétique (P) dans le fil à contrôler des première et deuxième branches (82, 84) définissant une direction d'alignement du dispositif,
- au moins un organe de réception (74) du flux magnétique (P1, P2 ; P) circulant dans le fil à contrôler (42a-b ; 100 ; 102) situé entre les première et deuxième branches (82, 84), dispositif dans lequel les dimensions de l'organe de réception (74) sont agencées de façon à mesurer seulement le flux magnétique circulant dans le fil à contrôler (42a-b ; 100 ; 102), lorsqu'en fonctionnement, la direction d'alignement du dispositif (18) est sensiblement parallèle à la direction selon laquelle s'étend le fil à contrôler (42a-b ; 100 ; 102).

2. Ensemble selon la revendication 1, dans lequel l'organe de réception (74) comprend une tête (86) de réception du flux présentant, dans la direction perpendiculaire à la direction d'alignement, une dimension sensiblement inférieure ou égale au diamètre du fil (42a-b ; 100 ; 102).

3. Ensemble selon la revendication 2, dans lequel la dimension de la tête de réception (86) dans la direction perpendiculaire à la direction d'alignement, est inférieure ou égale à 5 mm, voire 3 mm et de préférence 2 mm.

4. Ensemble selon l'une quelconque des revendications précédentes, dans lequel l'organe de réception (74) est situé à équidistance des première et deuxième branches (82, 84).

5. Ensemble selon l'une quelconque des revendications 1 à 4, dans lequel l'organe de réception (74) comprend un capteur magnétique du type magnéto-résistance, magnéto impédance ou à effet Hall.

6. Ensemble selon l'une quelconque des revendications 1 à 4, dans lequel le support porte des premier et deuxième organes (68, 70) d'induction d'un premier et deuxième flux magnétiques (P1, P2) dans le support magnétique (67) et dans le fil (42a-b ; 100 ; 102),

7. Ensemble selon la revendication 6, dans lequel chaque premier et deuxième organe d'induction (68, 70) comprend respectivement des première et deuxième bobines d'induction (80), chaque première et deuxième bobine (80) d'induction étant agencée autour respectivement des première et deuxième branches (82, 84).

8. Ensemble selon la revendication 6 ou 7, dans lequel l'organe de réception (74) comprend un noyau (86) autour duquel est agencée une bobine de réception (88) des premier et deuxième flux électromagnétiques (P1, P2), le noyau (86) formant la tête de réception des premier et deuxième flux magnétiques (P1, P2).

9. Procédé de contrôle d'un pneumatique comprenant des fils de renfort (42a-b ; 100 ; 102) de diamètre déterminé réalisés dans un matériau magnétique ou d'une nappe comprenant de tels fils de renfort (42a-b ; 100; 102) et disposés sensiblement parallèlement entre eux en faisant un angle donné avec la direction circonférentielle du pneumatique (14) ou avec la direction longitudinale de la nappe (98) au moyen d'un ensemble selon l'une quelconque des revendications 1 à 8, **caractérisé en ce qu'**on contrôle successivement chaque fil (42a-b ; 100 ; 102) du pneumatique (14) ou de la nappe (98) indépendamment les uns des autres.

10. Procédé selon la revendication 9, dans lequel l'organe de réception (74) comprend une tête (86) de réception du flux présentant, dans la direction perpendiculaire à la direction d'alignement, une dimension sensiblement inférieure ou égale au diamètre du fil (42a-b ; 100 ; 102).

11. Procédé selon la revendication 9 ou 10, dans lequel on déplace le pneumatique (14) ou la nappe (98) par rapport au dispositif (18) et on mesure un signal représentatif de l'état du ou de chaque fil (42a-b ; 100 ; 102) en fonction du déplacement relatif du pneumatique (14) ou de la nappe (98) par rapport au dispositif (18).

12. Procédé selon la revendication 11, dans lequel on compare la valeur du signal mesuré (E) à une valeur de référence (E₀) de sorte qu'on détecte un éventuel défaut du ou de chaque fil (42a-b ; 100 ; 102).

13. Procédé selon la revendication 12, dans lequel on détermine une caractéristique géométrique du ou de chaque fil (42a-b ; 100 ; 102) en fonction d'une variation du signal mesuré (E).

14. Procédé selon la revendication 13, dans lequel,
- on déplace circonférentiellement le pneumatique (14) ou la nappe (98) dans une direction longitudinale, par rapport au dispositif (18) respectivement sur une longueur circonférentielle donnée ou une longueur donnée
- on mesure la variation du signal (E) en fonction du déplacement du pneumatique (14) ou de la nappe (98),
- on détermine :
o un nombre de fils (42a-b ; 100 ; 102) dans la longueur circonférentielle du pneumatique (14) ou la longueur de la nappe (98), ou/et
o un pas entre chaque fil (42a-b ; 100 ; 102) dans la longueur circonférentielle du pneumatique (14) ou la longueur de la nappe (98).

15. Procédé selon la revendication 13, dans lequel,
- on déplace en rotation le dispositif (18) autour d'un axe sensiblement perpendiculaire à une surface, dite circonférentielle, du pneumatique (14) ou une surface, dite longitudinale, de la nappe (98),
- on mesure la variation du signal (E) en fonction de l'angle (α) de rotation du dispositif (18),
- on détermine l'angle formé par chaque fil (42a-b; 100; 102) avec la direction circonférentielle du pneumatique (14) ou avec la direction longitudinale de la nappe (98).

## Patentansprüche

1. Anordnung aus einem Reifen, der einen Verstärkungsdraht (42a-b; 100; 102) mit einem bestimmten Durchmesser aus einem magnetischen Material umfasst, und einer Vorrichtung (18) zur Überwachung dieses Verstärkungsdrahts, **dadurch gekennzeichnet, dass** die Vorrichtung umfasst:
- mindestens ein Glied (68, 70) zum Induzieren eines Magnetflusses (P1, P2; P) in dem zu überwachenden Draht (42a-b; 100; 102),
- einen Halter (67) aus magnetischem Material, der das Induktionsglied (68, 70) trägt, wobei der Halter (67) die Grundform eines U hat und erste und zweite Schenkel (82, 84) zum Leiten des Magnetflusses (P) in den zu überwachenden Draht (42a-b; 100; 102) umfasst, wobei die Enden des ersten und zweiten Schenkels (82, 84), die den Magnetfluss (P) in den zu überwachenden Draht leiten, eine Fluchtrichtung der Vorrichtung definieren,
- mindestens ein Glied (74) zum Empfangen des in dem zu überwachenden Draht (42a-b; 100; 102) fließenden Magnetflusses (P1, P2; P), das zwischen dem ersten und zweiten Schenkel (82, 84) liegt, wobei die Maße des Empfangsglieds (74) in der Vorrichtung so gestaltet sind, dass nur der in dem zu überwachenden Draht (42ab; 100; 102) fließende Magnetfluss gemessen wird, wenn während des Betriebs die Fluchtrichtung der Vorrichtung (18) annähernd parallel zu der Richtung ist, in der sich der zu überwachende Draht (42a-b; 100; 102) erstreckt.

2. Anordnung nach Anspruch 1, wobei das Empfangsglied (74) einen Kopf (86) zum Empfangen des Flusses umfasst, der in der Richtung senkrecht zur Fluchtrichtung ein Maß aufweist, das annähernd kleiner gleich dem Durchmesser des Drahtes (42a-b; 100; 102) ist.

3. Anordnung nach Anspruch 2, wobei das Maß des Empfangskopfes (86) in der Richtung senkrecht zur Fluchtrichtung gleich kleiner 5 mm oder sogar 3 mm und bevorzugt 2 mm ist.

4. Anordnung nach einem beliebigen der vorhergehenden Ansprüche, wobei das Empfangsglied (74) gleich weit vom ersten und zweiten Schenkel (82, 84) entfernt liegt.

5. Anordnung nach einem beliebigen der Ansprüche 1 bis 4, wobei das Empfangsglied (74) einen magnetischen Sensor von der Art eines Magnetwiderstandssensors, Magnetimpedanzsensors oder Halleffektsensors umfasst.

6. Anordnung nach einem beliebigen der Ansprüche 1 bis 4, wobei der Halter ein erstes und ein zweites Glied (68, 70) zum Induzieren eines ersten und eines zweiten Magnetflusses (P1, P2) im magnetischen Halter (67) und im Draht (42a-b; 100; 102) trägt.

7. Anordnung nach Anspruch 6, wobei jedes erste und zweite Induktionsglied (68, 70) eine erste bzw. eine zweite Induktionsspule (80) umfasst, wobei jede erste und zweite Induktionsspule (80) um den ersten bzw. zweiten Schenkel (82, 84) herum angeordnet ist.

8. Anordnung nach Anspruch 6 oder 7, wobei das Empfangsglied (74) einen Kern (86) umfasst, um den herum eine Spule (88) zum Empfangen des ersten und des zweiten elektromagnetischen Flusses (P1, P2) angeordnet ist, wobei der Kern (86) den Kopf zum Empfangen des ersten und des zweiten Magnetflusses (P1, P2) bildet.

9. Verfahren zur Überwachung eines Reifens mit Verstärkungsdrähten (42a-b; 100; 102) mit einem bestimmten Durchmesser aus einem magnetischen Material oder einer Lage, die solche Verstärkungsdrähte (42a-b; 100; 102) umfasst, die annähernd parallel zueinander angeordnet sind und einen gegebenen Winkel mit der Umfangsrichtung des Reifens (14) oder mit der Längsrichtung der Lage (98) bilden, mittels einer Anordnung nach einem beliebigen der Ansprüche 1 bis 8, **dadurch gekennzeichnet, dass** nacheinander jeder Draht (42a-b; 100; 102) des Reifens (14) oder der Lage (98) unabhängig von den anderen überwacht wird.

10. Verfahren nach Anspruch 9, wobei das Empfangsglied (74) einen Kopf (86) zum Empfangen des Flusses umfasst, der in der Richtung senkrecht zur Fluchtrichtung ein Maß aufweist, das annähernd kleiner gleich dem Durchmesser des Drahtes (42a-b; 100; 102) ist.

11. Verfahren nach Anspruch 9 oder 10, wobei der Reifen (14) oder die Lage (98) in Bezug auf die Vorrichtung (18) bewegt wird und ein Signal gemessen wird, das repräsentativ für den Zustand des oder jedes Drahtes (42a-b; 100; 102) ist, in Abhängigkeit von der relativen Bewegung des Reifens (14) oder der Lage (98) in Bezug auf die Vorrichtung (18).

12. Verfahren nach Anspruch 11, wobei der Wert des gemessenen Signals (E) mit einem Referenzwert (E₀) verglichen wird, so dass ein etwaiger Defekt des oder jedes Drahtes (42a-b; 100; 102) detektiert wird.

13. Verfahren nach Anspruch 12, wobei eine geometrische Eigenschaft des oder jedes Drahtes (42a-b; 100; 102) in Abhängigkeit von einer Änderung des gemessenen Signals (E) bestimmt wird.

14. Verfahren nach Anspruch 13, wobei
- der Reifen (14) oder die Lage (98) umfangsmäßig in einer Längsrichtung in Bezug auf die Vorrichtung (18) über eine gegebene Umfangslänge oder eine gegebene Länge bewegt wird,
- die Änderung des Signals (E) in Abhängigkeit von der Bewegung des Reifens (14) oder der Lage (98) gemessen wird,
- bestimmt wird:
o eine Anzahl von Drähten (42a-b; 100; 102) in der Umfangslänge des Reifens (14) oder der Länge der Lage (98) oder/und
o ein Abstand zwischen jedem Draht (42a-b; 100; 102) in der Umfangslänge des Reifens (14) oder der Länge der Lage (98).

15. Verfahren nach Anspruch 13, wobei
- die Vorrichtung (18) um eine Achse drehbewegt wird, die annähernd senkrecht zu einer Fläche, Umfangsfläche genannt, des Reifens (14) oder zu einer Fläche, Längsfläche genannt, der Lage (98) verläuft,
- die Änderung des Signals (E) in Abhängigkeit vom Drehwinkel (α) der Vorrichtung (18) gemessen wird,
- der Winkel bestimmt wird, der von jedem Draht (42a-b; 100; 102) mit der Umfangsrichtung des Reifens (14) oder mit der Längsrichtung der Lage (98) gebildet wird.

## Claims

1. Assembly of a tyre comprising a reinforcing thread (42a-b; 100; 102) of a given diameter made of a magnetic material and a device (18) for checking a tyre reinforcing thread (42a-b; 100; 102), **characterized in that** the device comprises:
- at least one induction member (68, 70) that induces a magnetic flux (P1, P2; P) in the thread that is to be checked (42a-b; 100; 102),
- a support (67) made of magnetic material supporting the induction member (68, 70), the support (67) having the overall shape of a U and comprising first and second legs (82, 84) that conduct the magnetic flux (P) into the thread for checking (42a-b; 100; 102), the ends for conducting the magnetic flux (P) into the thread for checking of the first and second legs (82, 84) defining a direction of alignment of the device,
- at least one receiver member (74) that receives the magnetic flux (P1, P2; P) flowing through the thread for checking (42a-b; 100; 102) located between the first and second legs (82, 84),
in which device the dimensions of the receiver member (74) are arranged so as to measure only the magnetic flux flowing through the thread for checking (42a-b; 100; 102) when, in operation, the direction of alignment of the device (18) is substantially parallel to the direction in which the thread for checking (42a-b; 100; 102) runs.

2. Assembly according to Claim 1, in which the receiver member (74) comprises a flux receiver head (86) which, in the direction perpendicular to the direction of alignment, has a dimension substantially smaller than or equal to the diameter of the thread (42a-b; 100; 102).

3. Assembly according to Claim 2, in which the dimension of the receiver head (86) in the direction perpendicular to the direction of alignment is less than or equal to 5 mm, or even 3 mm and preferably 2 mm.

4. Assembly according to any one of the preceding claims, in which the receiver member (74) is situated equal distances from the first and second legs (82, 84).

5. Assembly according to any one of Claims 1 to 4, in which the receiver member (74) comprises a magnetic sensor of the magneto-resistive sensor type, the magneto-impedance sensor type or the Hall-effect type.

6. Assembly according to any one of Claims 1 to 4, in which the support supports first and second induction members (68, 70) that induce a first and a second magnetic flux (P1, P2) in the magnetic support (67) and in the thread (42a-b; 100; 102).

7. Assembly according to Claim 6, in which each first and second induction member (68, 70) respectively comprises first and second induction coils (80), each first and second induction coil (80) being arranged respectively around the first and second legs (82, 84).

8. Assembly according to Claim 6 or 7, in which the receiver member (74) comprises a core (86) around which is arranged a receiver coil (88) that receives the first and second electromagnetic fluxes (P1, P2), the core (86) forming the receiver head that receives the first and second magnetic fluxes (P1, P2).

9. Method of monitoring or checking a tyre or a ply comprising reinforcing threads (42a-b; 100; 102) which are made of a magnetic material and laid substantially parallel to one another at a given angle to the circumferential direction of the tyre (14) or to the longitudinal direction of the ply (98), using a checking device (18) according to any one of Claims 1 to 8, **characterized in that** each thread (42a-b; 100; 102) of the tyre (14) or of the ply (98) is checked in succession independently of one another.

10. Method according to Claim 9, in which the receiver member (74) comprises a flux receiver head (86) which, in the direction perpendicular to the direction of alignment, has a dimension substantially smaller than or equal to the diameter of the thread (42a-b; 100; 102).

11. Method according to Claim 9 or 10, in which the tyre (14) or the ply (98) is moved with respect to the device (18) and a signal representative of the condition of the or each thread (42a-b; 100; 102) as a function of the relative movement of the tyre (14) or of the ply (98) with respect to the device (18) is measured.

12. Method according to Claim 11, in which the value of the measured signal (E) is compared against a reference value (E₀) so that any defect with the or each thread (42a-b; 100; 102) can be detected.

13. Method according to Claim 12, in which a geometric characteristic of the or each thread (42a-b; 100; 102) is determined as a function of a variation in the measured signal (E).

14. Method according to Claim 13, in which:
- the tyre (14) or the ply (98) is moved circumferentially in a longitudinal direction with respect to the device (18) over a given circumferential length or a given length, respectively,
- the variation in the signal (E) as a function of the movement of the tyre (14) or of the ply (98) is measured,
- the following are determined:
o a number of threads (42a-b; 100; 102) in the circumferential length of the tyre (14) or the length of the ply (98), and/or
o a spacing between each thread (42a-b; 100; 102) in the circumferential length of the tyre (14) or the length of the ply (98).

15. Method according to Claim 13, in which:
- the device (18) is rotated about an axis substantially perpendicular to a surface, called the circumferential surface, of the tyre (14) or a surface, called the longitudinal surface, of the ply (98),
- the variation in the signal (E) as a function of the angle (α) of rotation of the device (18) is measured,
the angle formed by each thread (42a-b; 100; 102) with the circumferential direction of the tyre (14) or with the longitudinal direction of the ply (98) is determined.
